# EUROPEAN PATENT SPECIFICATION

(11) **EP 4 301 296 B1**
(45) Date of publication and mention of the grant of the patent: **15.04.2026**
(21) Application number: 22711389.1
(22) Date of filing: 28.02.2022
(51) Int. Cl.: A61F 5/453, A61F 5/48

(54) **FLUID COLLECTION DEVICES HAVING A CONTINUOUS FLUID PERMEABLE BODY AND MULTIPLE ARMS, AND RELATED METHODS**
FLÜSSIGKEITSSAMMELVORRICHTUNGEN MIT EINEM DURCHGEHENDEN FLÜSSIGKEITSDURCHLÄSSIGEN KÖRPER UND MEHREREN ARMEN SOWIE ZUGEHÖRIGE VERFAHREN
DISPOSITIFS DE COLLECTE DE FLUIDE COMPORTANT UN CORPS CONTINU PERMÉABLE AUX FLUIDES ET DE MULTIPLES BRAS, ET PROCÉDÉS ASSOCIÉS

(30) Priority: 02.03.2021 US 202163155395 P
(43) Date of publication of application: 10.01.2024
(73) Proprietor: Purewick Corporation, El Cajon, California 92020 (US)
(72) Inventor: NEWTON, Camille Rose, Bonsall, California 92003 (US); AGRAWAL, Serena, Atlanta, Georgia 30306 (US)
(74) Representative: Hoffmann Eitle
(86) International application number: PCT/US2022/018170
(87) International publication number: WO 2022/187152

(56) References cited:
- US-A1- 2004 006 321
- US-B1- 6 248 096

## Description

### CROSS-REFERENCE TO RELATED APPLICATIONS

This application claims priority to U.S. Provisional Patent Application No. 63/155,395 filed on March 2, 2021.

### BACKGROUND

An individual may have limited or impaired mobility such that typical urination processes are challenging or impossible. For example, the individual may have surgery or a disability that impairs mobility. In another example, the individual may have restricted travel conditions such as those experienced by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, spills, and other hygiene issues. Urinary catheters may be uncomfortable, painful, and may cause urinary tract infections. US 2004/0006321 A1 discloses a urine collection device for a male, the device comprising a waterproof, thin-walled conduction tube surrounding the penis, having wettable internal walls and containing a spacing wick. At one end, the conduction tube is connected to, or surrounded by, an elastic compression tube that provides a uniform compressive force around the girth of the penis for attachment and liquid seal to said penis, and at the other end is attached to a fitting that connects to a conveyance tube. US 6,248,096 B1 discloses an apparatus for forming a fluid tight seal around a penis of a user comprising for controlling male urinary incontinence, the apparatus having a substantially fluid impermeable sheath having a proximal end and a distal end, an inner surface and an outer surface, wherein, in use, at least a portion of the inner surface contacts the skin of the penis.

Thus, users and manufacturers of fluid collection devices continue to seek new and improved devices, systems, and methods to collect urine.

### SUMMARY

Embodiments disclosed herein are related to fluid collection devices having a continuous fluid permeable body with multiple arms, and related systems and methods. According to a first aspect, there is provided a urine collection device according to claim 1.

According to a second aspect, there is provided a method of collecting urine according to claim 15.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1** is a block diagram of a fluid collection system, according to an embodiment.
**FIG. 2A** is a front isometric view of a urine collection device, according to an embodiment.
**FIG. 2B** is a front view of a distal end region of the fluid permeable body of the urine collection device of **FIG. 2A****,** according to an embodiment.
**FIG. 3** is a front isometric view of a distal end region of a urine collection device, according to an embodiment.
**FIG. 4** is a front view of a distal end region of a urine collection device, according to an embodiment.
**FIG. 5** is a cross-sectional side view of a distal end region of a urine collection device, according to an embodiment.
**FIG. 6A** is a front view of a distal end region of a urine collection device, according to an embodiment.
**FIG. 6B** is a front view of a distal end region of a urine collection device, according to an embodiment.
**FIG. 7A** is a front isometric view of a distal end region of a urine collection device, according to an embodiment.
**FIG. 7B** is a front isometric view of a distal end region of a urine collection device, according to an embodiment.
**FIG. 8A** is a front isometric view of a urine collection device, according to an embodiment.
**FIG. 8B** is a front isometric view of a urine collection device, according to an embodiment.
**FIG. 9** **is** a flow diagram of a method for collecting urine, according to an embodiment.

### DETAILED DESCRIPTION

Embodiments disclosed herein are related to fluid collection devices having a multiple arms in a fluid permeable body and/or a substantially continuous and uniform fluid permeable body. A fluid collection device may include a fluid impermeable barrier defining a chamber, a fluid permeable body positioned in the chamber, and a tube extending through the fluid impermeable barrier into the chamber. At least one, some, or all embodiments of fluid collection devices described herein include multiple arms or wings extending from a distal or common portion (*e.g.,* a central portion) of the fluid permeable body. At least a portion of the distal portion of the fluid permeable body may be positioned between the tube opening in the chamber and the distal end region of the fluid impermeable barrier. When an at least partial vacuum is applied to the tube, fluid in the chamber may be drawn through the multiple arms to a sump at the distal portion of the fluid permeable body for removal from the chamber through the tube. The multiple arms result in the technical effect of spreading the vacuum-assisted wicking action in more than one direction in the chamber. In at least one, some, or all embodiments of male urine collection devices described herein, multiple arms result in the technical effect of positioning the fluid permeable body to be positioned on both sides of the base of the penis in the chamber of the fluid collection device, even if the male is laying on his side while using the urine collection device. Urine discharged into the chamber, then, can either flow to the left or the right of the penis and be drawn into the arms of the fluid permeable body.

In at least one, some, or all embodiments described herein, the sump of the fluid collection device where fluid collects is positioned between the tube opening and a portion of the fluid impermeable barrier. At least a portion of the fluid permeable body may be positioned in the sump. The sump may be positioned at the lowest point of the fluid collection device during use, such as the distal end region of the fluid impermeable barrier. In some embodiments, the fluid collection device may be substantially horizontal, and the sump is positioned where fluid collects via vacuum assisted wicking action.

At least one, some or all embodiments described herein also include a substantially continuous and/or uniform fluid permeable body. The fluid permeable body results in the technical effect of providing a mechanism for liquid molecules to connect to each other. A substantially continuous and/or uniform fluid permeable body may connect arms or other portions of the fluid permeable body to the sump. Conventional urine collection devices often includes seams, gaps, or other interruptions to a uniform and/or continuous fluid permeable body. These seams, gaps, or other interruptions in the fluid permeable body disrupt the vacuum and/or flow of urine through the fluid permeable body.

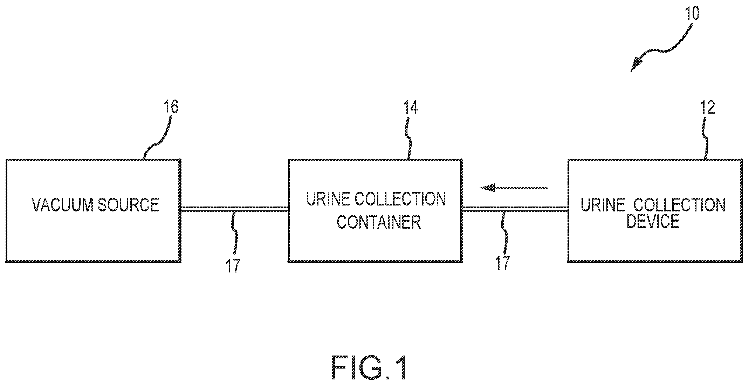

**FIG. 1** is a block diagram of a fluid collection system 10, according to an embodiment. The fluid collection system 10 may be included in any of the embodiments of fluid collection systems described herein. The system 10 includes a fluid (*e.g.,* urine) collection device 12 (*e.g.,* any of the fluid collection assemblies disclosed herein), a urine collection container 14, and a vacuum source 16 (or pump). The fluid collection device 12, the urine collection container 14, and the vacuum source 16 may be fluidly coupled to each other via one or more tubes 17 or conduits. For example, fluid collection device 12 may be operably coupled to one or more of the urine collection container 14 or the vacuum source 16 via the tube 17. In some embodiments, the vacuum source 16 may be secured directly to the urine collection container 14. Fluid (*e.g.,* urine or other bodily fluids) collected in the fluid collection device 12 may be removed from the fluid collection device 12 via the tube 17 secured to the fluid collection device 12. Suction force may be introduced into the chamber of the fluid collection device 12 via the inlet of the tube 17 responsive to suction (*e.g*., vacuum) force applied at the outlet of the tube 17.

The suction force may be applied to the outlet of the tube 17 by the vacuum source 16 either directly or indirectly. The suction force may be applied indirectly via the urine collection container 14. For example, the outlet of the tube 17 may be disposed within or fluidly coupled to an interior region of the urine collection container 14 and an additional tube 17 may extend from the urine collection container 14 to the vacuum source 16. Accordingly, the vacuum source 16 may apply suction to the fluid collection device 12 via the urine collection container 14. The suction force may be applied directly via the vacuum source 16. For example, the outlet of the tube 17 may be disposed within the vacuum source 16. An additional tube 17 may extend from the vacuum source 16 to a point outside of the fluid collection device 12, such as to the urine collection container 14. In such examples, the vacuum source 16 may be disposed between the fluid collection device 12 and the urine collection container 14.

The urine collection container 14 is sized and shaped to retain a fluid therein. The urine collection container 14 may include a bag (*e.g*., drainage bag), a bottle or cup (*e.g.,* collection jar), or any other enclosed container for storing bodily fluid(s) such as urine. In some examples, the tube 17 may extend from the fluid collection device 12 and attach to the urine collection container 14 at a first point therein. An additional tube 17 may attach to the urine collection container 14 at a second point thereon and may extend and attach to the vacuum source 16. Accordingly, a vacuum (*e.g*., suction) may be drawn through fluid collection device 12 via the urine collection container 14. Fluid, such as urine, may be drained from the fluid collection device 12 using the vacuum source 16.

The vacuum source 16 may include one or more of a manual vacuum pump, and electric vacuum pump, a diaphragm pump, a centrifugal pump, a displacement pump, a magnetically driven pump, a peristaltic pump, or any pump or other source configured to produce a vacuum (*e.g*., negative pressure). The pump 16 may provide a vacuum or suction to remove fluid from the fluid collection device 12. In some examples, the vacuum source 16 may be powered by one or more of a power cord (*e.g.,* connected to a power socket), one or more batteries, or even manual power (*e.g*., a hand operated vacuum pump). In some examples, the vacuum source 16 may be sized and shaped to fit outside of, on, or within the fluid collection device 12. For example, the vacuum source 16 may include one or more miniaturized pumps or one or more micro pumps. The vacuum sources disclosed herein may include one or more of a switch, a button, a plug, a remote, or any other device suitable to activate the vacuum source 16.

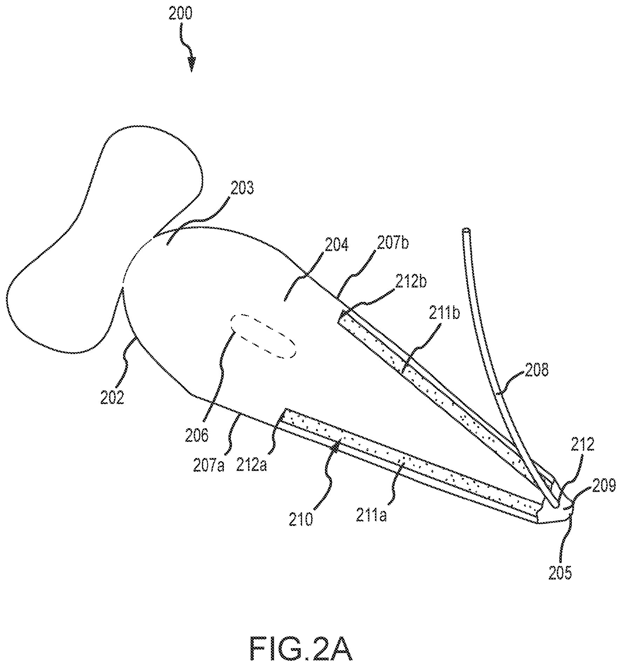

**FIG. 2A** is a front view of a male urine collection device 200, according to an embodiment. The urine collection device 200 includes a fluid impermeable barrier 202 at least partially defining an opening 206 and a chamber 204 within the fluid collection device in fluid communication with the opening 206. In **FIG. 2A****,** the opening 206 is on a bottom layer of the fluid impermeable barrier 202 underneath a top layer of the fluid impermeable barrier 202 when the urine collection device is in use. The fluid impermeable barrier includes a proximal end region 203 and a distal end region 205 which may at least partially define the chamber 204 therein. The opening 206 may be positioned proximate or closer to the proximal end region 203 than the distal end region 205 of the fluid impermeable barrier 202. For example, the opening 206 may be positioned less than about halfway, less than about one-third, or less than about one-quarter of a distance from the proximal end region 203 to the distal end region 205. In some embodiments, the opening 206 may be positioned proximate or closer to the distal end region 205 than the proximal end region 203 of the fluid impermeable barrier 202. For example, the opening 206 may be positioned less than about halfway, less than about one-third, or less than about one-quarter of a distance from the distal end region 205 to the proximal end region 203.

In some embodiments, the fluid impermeable barrier 202 narrows between the proximal end region 203 and the distal end region 205. For example, the fluid impermeable barrier 202 may include a substantially triangular front profile, with the distal end region 205 being at the narrow end or tip of the triangular profile. The fluid impermeable barrier 202 may include edges 207a, 207b that extend at least partially between the proximal end region 203 and the distal end region 205 of the fluid impermeable barrier. The chamber 204 may include a shape substantially complementary to the fluid impermeable barrier 202. The fluid impermeable barrier 202 may include a substantially flexible fluid impermeable material, such as a fluid impermeable polymer (*e.g*., silicone, polypropylene, polyethylene, polyethylene terephthalate, a polycarbonate, etc.), polyurethane films, thermoplastic elastomer, oil, another suitable material, or combinations thereof. In some embodiments, the fluid impermeable barrier 202 includes a paper-like fluid impermeable material or a fluid impermeable fabric.

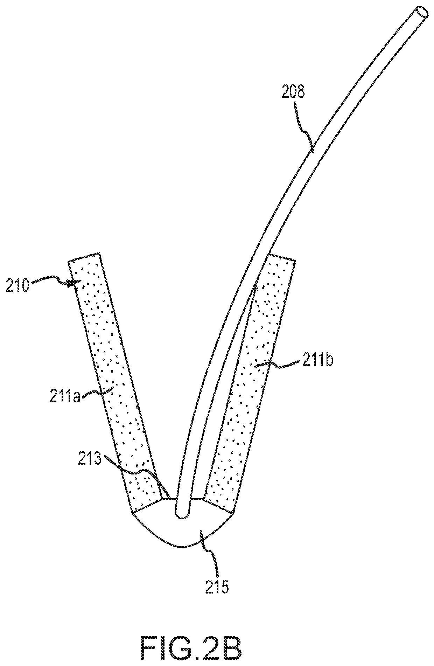

The urine collection device 200 also includes a fluid permeable body 210 positioned within the chamber 204 and extending at least partially between the distal end region 205 and the proximal end region 203. In some embodiments, the fluid permeable body 210 includes a distal portion 213 (not visible in **FIG. 2A****,** shown in **FIG. 2B****)** positioned in the distal end region 205 of the fluid impermeable barrier 202, and the fluid permeable body 210 extends from the distal end region 205 of the chamber 204 towards the opening 206. The fluid permeable body 210 may include multiple (*e.g*., two, three, four, five, six, or more) arms that combine at the distal portion 213 of the fluid permeable body positioned at the distal end region 205 to form a sump 209 in the chamber 204. For example, the fluid permeable body 210 include a first arm 211a extending from the distal portion 213 of the fluid permeable body 210 towards the proximal end region 203 of the fluid impermeable barrier 202, and a second arm 211b spaced from the first arm 211a and extending from the distal portion 213 of the fluid permeable body 210 towards the proximal end region 203 of the fluid impermeable barrier 202. In some embodiments, the fluid permeable body 210 may include one or more additional arms positioned between the first arm 211a and the second arm 211b, each of the one or more additional arms extending from the distal portion 213 of the fluid permeable barrier 210 towards the opening 206 and/or the proximal end region 203 of the fluid impermeable barrier 202. In some embodiments, the first arm 211a is positioned in the chamber 204 at least proximate or adjacent to (*e.g.,* abutting) at least a portion of the first edge 207a of the fluid impermeable barrier 202, and the second arm 211b is positioned in the chamber 204 proximate or adjacent to (*e.g.,* abutting) at least a portion of the second edge 207b of the fluid impermeable barrier 202.

The fluid permeable body 210 can be configured to wick and/or allow transport of fluid away from the opening 206, thereby preventing the fluid from escaping the chamber 204. The fluid permeable body 210 also can wick and/or allow transport of the fluid generally towards the sump 209. The fluid permeable body 210 can include any material that can wick and/or allow transport of the fluid. For example, material(s) and configurations of the fluid permeable body 210 described herein provide a texture, depth, width, and hydrophilicity that enhances liquid flow through the fluid permeabley body 210 to the tube 208. The permeable properties referred to herein can be wicking, capillary action, diffusion, or other similar properties or processes, and are referred to herein as "permeable" and/or "wicking." Such "wicking" or other physical properties may exclude absorption into the fluid permeable body 210, such as not include adsorption of the bodily fluids into the fluid permeable body 210. Put another way, substantially no absorption or solubility of the bodily fluids into the material may take place after the material is exposed to the bodily fluids and removed from the bodily fluids for a time. While no absorption or solubility is desired, the term "substantially no absorption" may allow for nominal amounts of absorption and/or solubility of the bodily fluids into the fluid permeable body 210 (*e.g.,* absorbency), such as less than about 30 wt% of the dry weight of the fluid permeable body 210, less than about 20 wt%, less than about 10 wt%, less than about 7 wt%, less than about 5 wt%, less than about 3 wt%, less than about 2 wt%, less than about 1 wt%, or less than about 0.5 wt% of the dry weight of the fluid permeable body 210. In an embodiment, the fluid permeable body 210 may include at least one absorbent or adsorbent material.

The fluid permeable body 210 can include a one-way fluid movement fabric. As such, the fluid permeable body 210 can remove fluid from the area around the penis, thereby leaving the area and urethra dry. The fluid permeable body 210 can enable the fluid to flow generally towards the sump 209 and the tube 208 within the chamber 204. The fluid permeable body 210 can include a porous or fibrous material, such as hydrophilic polyolefin. In some embodiments, the fluid permeable body 210 consists of or consists essentially of a porous or fibrous material, such as hydrophilic polyolefin. Examples of polyolefin that can be used in the fluid permeable body 210 include, but are not limited to, polyethylene, polypropylene, polyisobutylene, ethylene propylene rubber, ethylene propylene diene monomer, or combinations thereof. Moreover, the fluid permeable body 210 can be manufactured according to various manufacturing methods, such as molding, extrusion, or sintering. The fluid permeable body 210 can include varying densities or dimensions.

In some embodiments, any portion of the fluid permeable body 210 can include two or more layers of fluid permeable materials. For example, the fluid permeable body 210 can include a fluid permeable membrane covering or wrapped around a fluid permeable support, with both the fluid permeable membrane and the fluid permeable support being disposed in the chamber 204. The fluid permeable membrane can cover or extend across at least a portion (*e.g.,* all) of at least the side of the fluid permeable support facing the penis of the user. The fluid permeable membrane and the fluid permeable support can be configured to wick any fluid away from the opening 206, thereby preventing the fluid from escaping the chamber 204 and promoting removal of the fluid through the tube 208. The permeable properties referred to herein can be wicking, capillary action, diffusion, or other similar properties or processes, and are referred to herein as "permeable" and/or "wicking."

The fluid permeable membrane and the fluid permeable support also can wick and/or allow transport of the fluid generally towards the distal end region 205 the chamber 204, where the urine may collect in a sump 209 for remove through the tube 208. The fluid permeable membrane can include any material that can wick the fluid. For example, the fluid permeable membrane can include fabric, such as a gauze (*e.g.,* a silk, linen, polymer based materials such as polyester, or cotton gauze), nylon (such as a spun nylon fibers), another soft fabric (*e.g.,* jersey knit fabric or the like), another smooth fabric (*e.g.,* rayon, satin, or the like), a paper-based wicking material, or bamboo. Forming the fluid permeable membrane from gauze, soft fabric, and/or smooth fabric can reduce chaffing caused by the urine collection device 200. The wicking capability of the fluid permeable membrane also draws fluid into the fluid permeable body 210 and limits or reduces vacuum loss from exposed portion of the fluid permeable body. Other embodiments of fluid permeable membranes and fluid permeable supports are disclosed in U.S. Patent Application No. 15/612,325 filed on June 2, 2017; U.S. Patent Application No. 15/260,103 filed on September 8, 2016; U.S. Patent Application No. 15/611,587 filed on June 1, 2017; PCT Patent Application No. PCT/US19/29608, filed on April 29, 2019. In many embodiments, any portion of the fluid permeable body 210 (*e.g.,* the arms 211a, 211b and/or the distal portion 213) includes a fluid permeable support including a porous spun nylon fiber structure and a fluid permeable wicking membrane including gauze. For example, the fluid permeable body 210 may include a gauze or other wicking fabric positioned to contact the skin of the user. In some embodiments, the gauze or other wicking fabric is wrapped around a cylindrically formed spun nylon fibers material and/or covering both sides of a substantially planar spun nylon fibers material. In some embodiments, the gauze or other wicking fabric covers the side of substantially planar spun nylon fibers material that is oriented towards the skin of the user. In some embodiments, the fluid permeable body 210 may include an additional material and/or layer on top of the gauze or other wicking fabric. For example, the fluid permeable body 210 may include a hydrophobic web positioned on the gauze or other wicking fabric such that the penis rests on the hydrophobic web when the urine collection device 200 is in use. The urine collection device 200, then, may include the hydrophobic web as a top or outer layer, the gauze or other wicking material as an intermediate layer, and the spun nylon fibers or spun plastic as the bottom layer that provides the structure for molecular bonds of the urine in the fluid permeable body 210.

In at least one, some, or all embodiments described herein, the multiple arms 211a, 211b result in the technical effect of the fluid permeable body 210 being positioned on both sides of the base of the penis when the penis is positioned in the chamber 204, even if the male is laying on his side while using the urine collection device 200. For example, the arms 211a, 211b may extend from the distal portion 213 (at least proximate to the distal end region 205 of the fluid impermeable barrier 202) at least partially around opposing sides of the penis in the chamber 204. The arms 211a, 211b of the fluid permeable body may extend only partially from the distal portion 213 in the distal end region 205 to the proximal end region 203. In some embodiments, the arms 211a, 211b extend from the distal portion 213 to a terminal portion having a terminating edge 212a, 212b. In some embodiments, the arms 211a, 211b extend from the distal portion 213 to the terminating edges 212a, 212b such that the terminating edges 212a, 212b are generally aligned or level with a proximal edge of the opening 206 or a distal edge of the opening 206. In some embodiments, the arms 211a, 211b extend at least about one-half or at least about three-quarters of a distance from the distal end region 205 to the opening 206. In some embodiments, the arms 211a, 211b extend beyond the opening 206 and interface one another between the opening 206 and the proximal portion 203 of the fluid impermeable barrier 202. Each of the arms 211a, 211b may include a lateral width or diameter of at least about 0.5 cm, at least about 1 cm, at least about 2 cm, at least about 3 cm, at least about 4 cm, at least about 5 cm, about 0.5 cm to about 5 cm, about 1 cm to about 2 cm, about 2 cm to about 3 cm, about 3 cm to about 4 cm, or about 4 cm to about 5 cm.

In some embodiments, the fluid permeable body 210 is generally continuous and/or uniform from the terminating ends 212a, 212b to the distal portion 213 in the sump 209 and/or the distal end region 205 of the fluid impermeable barrier 202. For example, the material(s) and thickness of the fluid permeable body 210 may be substantially uniform from the terminating ends 212a, 212b to (and throughout) the distal portion 213. In some embodiments, the fluid permeable body 210 may include a continuous substantially planar surface on the first arm 211a, the second arm 211b, and the distal portion 213. In some embodiments, the first arm 211a and the second arm 211b may be generally cylindrical.

The arms 211a, 211b may be angled in the chamber 204 relative to one another. For example, the arms 211a, 211b may be angled relative to another about 1 degree to about 180 degrees, about 1 degree to about 15 degrees, about 15 degrees to about 30 degrees, about 30 degrees to about 45 degrees, about 45 degrees to about 60 degrees, about 60 degrees to about 75 degrees, about 75 degrees to about 90 degrees, about 90 degrees to about 105 degrees, about 105 degrees to about 120 degrees, about 120 degrees to about 135 degrees, about 135 degrees to about 140 degrees, about 140 degrees to about 155 degrees, about 155 degrees to about 170 degrees, or about 170 degrees to about 180 degrees.

At least the first arm 211a and the second arm 211b of the fluid permeable body 210 may include fluid permeable porous fiber structure and an outer fluid permeable wicking material. For example, the outer fluid permeable wicking material may be positioned in the chamber to interface the top portion or layer of the fluid impermeable barrier 202 and/or contact the skin of the penis inserted into the chamber 204. In some embodiments, the outer fluid permeable wicking material surrounds the fluid permeable porous fiber structure such that the fluid permeable porous fiber structure may be wrapped in the fluid permeable wicking material. The outer fluid permeable wicking material, then, may interface the bottom portion or layer of the fluid impermeable barrier 202 in some embodiments.

The fluid permeable body 210 may include a substantially uniform thickness from the terminating ends 212a, 212b to at least the distal portion 213 of the fluid permeable body 210 positioned at least proximate to the distal end region 205 of the fluid impermeable barrier 202. The thickness of the fluid permeable body 210 may be a dimension of the fluid permeable body 210 as measured between the top portion of the fluid permeable barrier 202 and the bottom portion of the fluid permeable barrier 202. In some embodiments, the fluid permeable body 210 may include a thickness of at least about 3 mm, at least about 4 mm, at least about 5 mm, at least about 6 mm, at least about 7 mm, at least about 8 mm, at least about 9 mm, at least about 1 cm, at least about 1.25 cm, at least about 1.5 cm, at least about 1.75 cm, at least about 2 cm, at least about 2.5 cm, at least about 3 cm, at least about 3.5 cm, about 3 mm to about 3 cm, about 4 mm to about 6 mm, about 6 mm to about 8 mm, about 8 mm to about 1 cm, about 1 cm to about 1.25 cm, about 1.25 cm to about 1.5 cm, about 1.5 cm to about 1.75 cm, or about 1.75 cm to about 2 cm, about 2 cm to about 2.25 cm, about 2.25 cm to about 2.5 cm, about 2.5 cm to about 2.75 cm, about 2.75 cm to about 3 cm, about 3 cm to about 3.25 cm, about 3.25 cm to about 3.5 cm, about 3.5 cm to about 3.75 cm, or about 3.75 cm to about 4 cm.

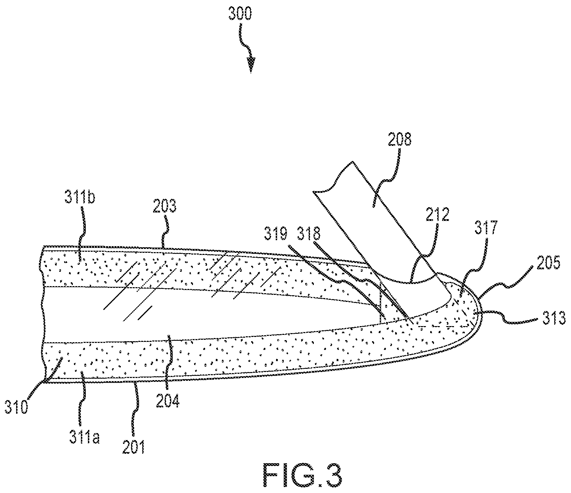

The urine collection device 200 also includes a tube 208 extending into the chamber 204 and having an end positioned proximate to the distal end region 205 of the fluid impermeable barrier 202. The tube 208 includes a tube opening or inlet proximate to the end of the tube 208 that is positioned in the chamber 204 (*e.g.,* the tube opening or inlet is in fluid communication with the chamber 204). A sump 209 may be defined as the region or area between the tube opening and the portion of the distal end region 205 of the fluid impermeable barrier 202. The sump 209 may be generally devoid of materials except portion(s) of the fluid permeable body 210. At least some of the fluid permeable body 210 may extend into the sump 209, such as the distal portion 213 (shown in **FIGS. 3****,** **5****,** **6A****,** and **6B**)**.** Fluid discharged on and/or pulled into the fluid permeable body 210 may flow to and pool in the sump 209 for removal when a vacuum is applied on the tube 208. The tube 208 may extend through an aperture 212 in a top layer or portion of the fluid impermeable barrier 202 and into the chamber 204. The aperture 212 may be positioned proximate to the distal end region 205 of the fluid impermeable barrier 202. In some embodiments, the end of the tube 208 and/or the tube opening are positioned between the distal end region 205 and the aperture 212. In some embodiments, the aperture 212 is absent, and the tube 208 may exit the chamber 204 through the opening 206. U.S. Provisional Patent 63/067,542 describes embodiments of male external catheter urine collection devices, aspects of which may be used in any of the embodiments disclosed herein.

Although the urine collection device 200 is described as configured for use in collecting urine, aspects of the urine collection device may be configured as fluid collection devices that collect blood, sweat, wound drainage, or other bodily fluids. For example, any aspect of the fluid permeable body 210 may be at least partially enclosed in a fluid collection device having an impermeable barrier defining an opening disposed on the fluid collection device to be positioned against the skin of the user. In this configuration, the gauze or other wicking material may be positioned to interface the skin of the user, with the gauze or other wicking material positioned between the spun fibers material and the skin. A vacuum tube may contact a portion of the fluid permeable body 210 to withdraw fluid from the fluid collection device.

**FIG. 2B** is a front view of the urine collection device 200 of **FIG. 2A** without the fluid impermeable barrier 202. In some embodiments, the urine collection device 200 includes a fluid impermeable membrane 215 or tape covering at least the distal portion 213 of the fluid permeable body 210. The fluid impermeable membrane 215 may be positioned in the chamber 204 adjacent to the distal end region 205 of the fluid impermeable barrier 202, and the tube 208 may extend through both the fluid impermeable barrier 202 and the fluid impermeable membrane 215. In some embodiments, each of the arms 211a, 211b include the fluid impermeable membrane 215 secured to the arms 211a, 211b between the bottom portion of the fluid impermeable barrier and the arms 211a, 211b. The fluid impermeable membrane 215 may include a waterproof tape such as a polyvinyl chloride foam tape with acrylate adhesive.

The distal portion 213 of the fluid permeable body 210 is shown covered by the fluid impermeable membrane 215 in **FIG. 2B****.** In other embodiments, the fluid impermeable membrane 215 is absent (*e.g*., there is no fluid impermeable membrane 215 covering the distal portion 213 of the fluid permeable body 210 positioned in the distal end region 205 of the fluid impermeable barrier 202). The distal portion 213 of the fluid permeable body 210 may include at least a fluid permeable porous fiber structure. The fluid permeable porous fiber structure of the distal portion 213 may be integral, continuous, and/or uniform with the fluid permeable fiber structure of the arms 211a, 211b of the fluid permeable body 210. In some embodiments, the distal portion 213 of the fluid permeable body 210 also includes an outer fluid permeable wicking material. The outer fluid permeable wicking material of the distal portion 213 of the fluid permeable body 210 may be integral, continuous, and/or uniform with the outer fluid permeable wicking material of the arms 211a, 211b of the fluid permeable body 210. The outer fluid permeable wicking material may be positioned on the distal portion of the fluid permeable body 210 to interface the top portion or layer of the distal end region 205 of fluid impermeable barrier 202. In some embodiments, the outer fluid permeable wicking material surrounds the fluid permeable porous fiber structure such that the fluid permeable porous fiber structure at the distal portion 213 may be wrapped in the fluid permeable wicking material. The outer fluid permeable wicking material, then, may interface the bottom portion or layer of the distal end region 205 of the fluid impermeable barrier 202 in some embodiments. The distal portion 213 of the fluid permeable body 210 may, in some embodiments, include only the fluid permeable porous fiber structure, and the fluid permeable wicking material may be absent from the distal portion 213 of the fluid permeable body 210.

In some embodiments, the distal portion 213 extends in the chamber 204 only partially from the distal end region 205 of the fluid impermeable barrier 202. For example, the distal portion 213 may extend in the chamber 204 from the distal end region 205 of the fluid impermeable barrier 202 less than about one-half, less than about one-third, less than about one-quarter, less than about one-fifth, less than about one-sixth, less than about one-seventh, less than about one-eighth, less than about one-ninth, or less than about one-tenth of a distance from the distal end region 205 of the fluid impermeable barrier 202 to the opening 206.

**FIG. 3** is a front isometric view of a distal end region of a urine collection device 300. Unless otherwise noted, the urine collection device 300 may include any aspect of the urine collection device 200. For example, the urine collection device 300 may include a fluid impermeable barrier 202 defining a chamber 204, an aperture 212, and an opening (not shown), and having a proximal end region (not shown), a distal end region 205, a bottom layer or portion 201, and a top layer or portion 203.

The urine collection device 300 includes a fluid permeable body 310 having a distal portion 313 and two or more arms 311a, 311b extending from the distal portion 313. Unless otherwise noted, the fluid permeable body 310 may include any aspect of the fluid permeable body 210, such as materials, dimensions, uniformity, and continuity. For example, each of the arms 311a, 311b and the distal portion 313 may include an outer fluid permeable wicking material and a fluid permeable porous fiber structure. The fluid permeable body of the arms 311a, 311b and the distal portion 313 may be substantially uniform and continuous from terminating ends of the arms 311a, 311b to the distal portion 313 of the fluid permeable body 310. In some embodiments, the arms 311a, 311b may be at least partially or substantially cylindrical.

The distal portion 313 of the fluid permeable body 310 may form a protrusion 317 or raised portion and a base 319. The base 319 and the protrusion 317 may include any aspect of the fluid permeable body 310, including the fluid permeable materials. A pocket 318 may be defined between the protrusion 317 and the base 319 of the distal portion 313. The pocket 318 may be sized to hold a portion of the tube 208 therein, including the end of the tube 208 and the tube opening on the tube 208.

In some embodiments, the end of the tube 208 includes a beveled end with the tube opening positioned at the beveled end. The beveled end of the tube 208 may be at an acute or obtuse angle relative to the sidewall of the tube 208. With the beveled end being angled relative to the sidewall of the tube 208, when the beveled end and the tube opening are generally horizontal, the tube 208 may angle relative to the base 319 of the distal portion 313 of the fluid permeable body 310 and/or the bottom portion 201 of the fluid impermeable barrier 202. For example, the tube 208 may angle from the beveled end towards the aperture 212 and out of the chamber 204.

Embodiments having a beveled end result in the technical effect of more efficient use of the urine collection device 300 when the urine collection device 300 is positioned generally horizontal on a user. In operation, when a vacuum is applied to the tube 208, the tube 208 draws fluid from the sump into the tube 208 through the tube opening. The fluid permeable body 310 provides connections for molecular bonding of water molecules in urine in the chamber 204. The urine being drawn into the tube 208 through the tube opening, then, also may draw urine in the fluid permeable body 210 to the sump via the molecular bonding of the water in the urine. The configuration of the sump within the urine collection device 300 may prevent a loss of vacuum on fluid being drawn from the urine collection device 300, thereby improving fluid capture from the fluid collection device. In some embodiments, the distal end region 205 of the fluid impermeable barrier 202 may be the low point of the urine collection device 300 during use, and the tube 208 may include an end that is generally perpendicular to the sidewall of the tube 208. In some embodiments, the distal end region 205 of the fluid impermeable barrier 202 may be horizontal or positioned at least partially uphill from other portions of the urine collection device 300. When the distal end region 205 of the fluid impermeable barrier 202 is horizontal or positioned at least partially uphill from other portions of the urine collection device 300, the vacuum applied to the tube 208 may draw urine in the fluid permeable body 210 to the tube 208 for removal.

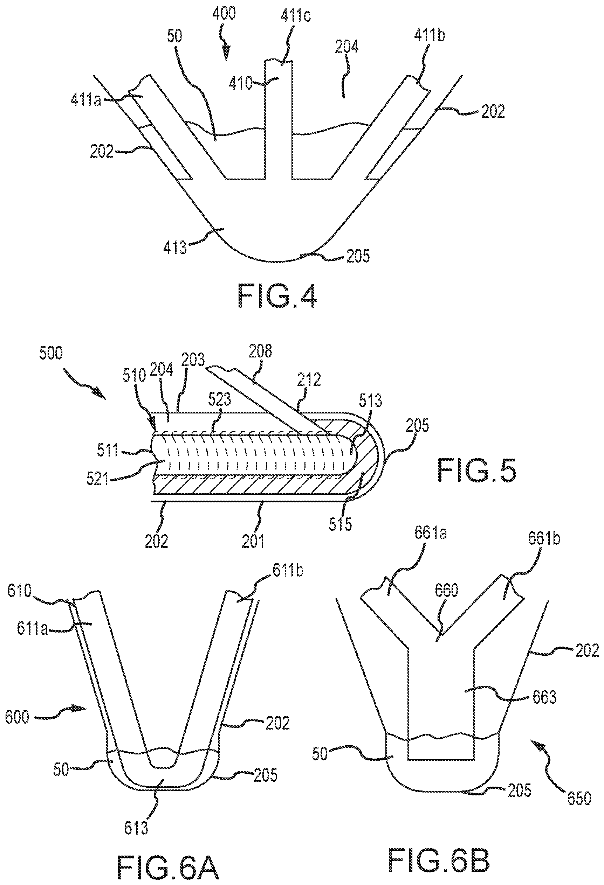

**FIG. 4** is a front view of a distal end region of a urine collection device 400 with urine 50 being held in the distal end region 205 of the fluid impermeable barrier 202. Unless otherwise noted, the urine collection device 400 may include any aspect of the urine collection devices 200, 300. For example, the urine collection device 400 may include the fluid impermeable barrier 202 defining a chamber, an aperture, and an opening (not shown), and having a proximal end region (not shown) and the distal end region 205. The tube 208 is not shown in **FIG. 4** to allow an unobstructed view of a fluid permeable body 410, but may include any aspect of the tube 208 shown and described in the urine collection devices 200, 300.

The urine collection device 400 includes the fluid permeable body 410 having a distal portion 413 and three or more arms 411a, 411b, 411c extending from the distal portion 413. Unless otherwise noted, the fluid permeable body 410 may include any aspect of the fluid permeable bodies 210, 310 such as materials, dimensions, uniformity, and continuity. For example, each of the arms 411a, 411b, 411c and the distal portion 413 may include an outer fluid permeable wicking material and a fluid permeable porous fiber structure. The fluid permeable body of the arms 411a, 411b, 411c and the distal portion 413 may be substantially uniform and continuous from terminating ends of the arms 411a, 411b, 411c, to the distal portion 413 of the fluid permeable body 410.

While the urine collection devices 200, 300 include fluid permeable bodies 210, 310 having two arms, some embodiments may include a fluid permeably body having three or more arms. The fluid permeable body 410, for example, has a first arm 411a, a second arm 411b, and a third arm 411c positioned between the first arm 411a and the second arm 411b. Other embodiments may include four, five, six, or more arms extending from the distal portion 413 of the fluid permeable body 410. The arms 411a, 411b, 411c may include any aspect of the arms of the fluid permeable bodies 210, 310 described above, such as materials, dimensions, uniformity, positioning, and continuity. For example, the arms 411a, 411b, 411c may include a fluid permeable porous fiber structure and an outer fluid permeable wicking material. In some embodiments, the arms 411a, 411b, 411c are substantially planar or substantially cylindrical.

While the arms 411a, 411b, 411c are angled relative to one another, in some embodiments, the urine collection device 400 may include multiple arms 411a, 411b, 411c that are substantially parallel to on another. Arms 411a, 411b, 411c that are substantially parallel to another may be spaced from one another or may abut/interface adjacent arm(s) of the arms 411a, 411b, 411c. In some embodiments, the urine collection device 400 may include three, four, five, or more arms 411 substantially parallel to one another. In some embodiments, each of the arms 411a, 411b, 411c may include a spun nylon fibers material, and a single sheet of wicking material (such as gauze) may overlay all of the arms 411a, 411b, 411c.

**FIG. 5** is a side cross-sectional view of a distal end region of a urine collection device 500. Unless otherwise noted, the urine collection device 500 may include any aspect of the urine collection devices 200, 300, 400. For example, the urine collection device 500 may include the fluid impermeable barrier 202 defining a chamber 204, an aperture 212, and an opening (not shown), and having a proximal end region (not shown) and the distal end region 205.

The urine collection device 500 also includes fluid permeable body 510 having a distal portion 513 and multiples (*e.g*., two, three, four, five, six, or more) arms 511 extending from the distal portion 513. Unless otherwise noted, the fluid permeable body 510 may include any aspect of the fluid permeable bodies 210, 310, 410 such as materials, dimensions, uniformity, and continuity. For example, the arms 511and the distal portion 513 may include an outer fluid permeable wicking material 523 and a fluid permeable porous fiber structure 521. In some embodiments, the outer fluid permeable wicking material 523 surrounds the fluid permeable porous fiber structure 521 such that the fluid permeable porous fiber structure 521 may be wrapped in the fluid permeable wicking material 523. The fluid permeable body of other urine collection devices described herein may include the fluid permeable body 510 of the urine collection device 500.

In some embodiments, the urine collection device 500 includes a fluid impermeable membrane 515 or tape covering at least the distal portion 513 of the fluid permeable body 510. The fluid impermeable membrane 515 may include a waterproof tape such as a polyvinyl chloride foam tape with acrylate adhesive. The fluid impermeable membrane 515 may be positioned in the chamber 204 adjacent to the distal end region 205 of the fluid impermeable barrier 202, and the tube 208 may extend through both the fluid impermeable barrier 202 and the fluid impermeable membrane 515. In some embodiments, each of the arms 511 include the fluid impermeable membrane 515 secured to the arms 515 between the bottom portion 201 of the fluid impermeable barrier 202 and the arms 511. The fluid impermeable membrane 515 may be positioned on the bottom of the arms 511 and extend from the distal portion 513 to the terminating ends of the arms 511. In some embodiments, the fluid impermeable membrane 515 is positioned on at least a portion of the bottom of the arms 511 and extends around the distal portion 513 to the top of the fluid permeable body 210 until the fluid permeable membrane 515 meets the tube 208. In some embodiments, the fluid impermeable barrier 202 is absent, and the fluid impermeable membrane 515 is positioned on and/or adhered to the skin of the user such that the arms 511 direct discharged urine to the distal portion 513 for removal. In other embodiments of the urine collection device 500, the fluid impermeable membrane 515 is absent.

**FIG. 6A** is a front view of a distal end region of a urine collection device 600 with urine 50 being held in the distal end region 205 of the fluid impermeable barrier 202. Unless otherwise noted, the urine collection device 600 may include any aspect of the urine collection devices 200, 300, 400, 500. For example, the urine collection device 600 may include the fluid impermeable barrier 202 defining a chamber, an aperture, and an opening (not shown), and having a proximal end region (not shown) and the distal end region 205. The tube 208 is not shown in **FIG. 6A** to allow an unobstructed view of a fluid permeable body 610, but may include any aspect of the tube 208 shown and described in the urine collection devices 200, 300.

The urine collection device 600 includes a V- or U-shaped fluid permeable body 610 having a distal portion 613 and two or more arms 611a, 611b extending from the distal portion 613. Unless otherwise noted, the fluid permeable body 610 may include any aspect of the fluid permeable bodies 210, 310, 410, 510, such as materials, dimensions, uniformity, and continuity. For example, each of the arms 611a, 611b and the distal portion 613 may include an outer fluid permeable wicking material and a fluid permeable porous fiber structure. The fluid permeable body 610 of the arms 611a, 611b and the distal portion 613 may be substantially uniform and continuous from terminating ends of the arms 611a, 611b to the distal portion 613 of the fluid permeable body 610.

**FIG. 6B** is a front view of a distal end region of a urine collection device 650 with urine 50 being held in the distal end region 205 of the fluid impermeable barrier 202. Unless otherwise noted, the urine collection device 650 may include any aspect of the urine collection devices 200, 300, 400, 500. For example, the urine collection device 650 may include the fluid impermeable barrier 202 defining a chamber, an aperture, and an opening (not shown), and having a proximal end region (not shown) and the distal end region 205. The tube 208 is not shown in **FIG. 6B** to allow an unobstructed view of a fluid permeable body 660, but may include any aspect of the tube 208 shown and described in the urine collection devices 200, 300.

The urine collection device 650 includes a Y-shaped fluid permeable body 610 having a distal portion 663 and two or more arms 661a, 661b extending from the distal portion 663. Unless otherwise noted, the fluid permeable body 660 may include any aspect of the fluid permeable bodies 210, 310, 410, 510, such as materials, dimensions, uniformity, and continuity. For example, each of the arms 661a, 661b and the distal portion 663 may include an outer fluid permeable wicking material and a fluid permeable porous fiber structure. The fluid permeable body 660 of the arms 661a, 661b and the distal portion 663 may be substantially uniform and continuous from terminating ends of the arms 661a, 661b to the distal portion 663 of the fluid permeable body 660. In some embodiments, the width of the arms 661a, 661b may decrease as the arms 661a, 661b closer to the distal portion 663.

In some embodiments, the distal portion 663 may be elongated from the distal end region 205 towards the proximal end region of the fluid impermeable barrier 202. The arms 661a, 66b1b may extend from a first end of the elongated distal portion 663, and the distal portion 663 may extend from the arms 661a, 661b to an opposite end of the distal portion 663 positioned at the distal end region 205 of the fluid impermeable barrier 202. In some embodiments, the fluid collection device 650 may include more than the two arms 661a, 661b shown, such as three, four, five, six, or more arms.

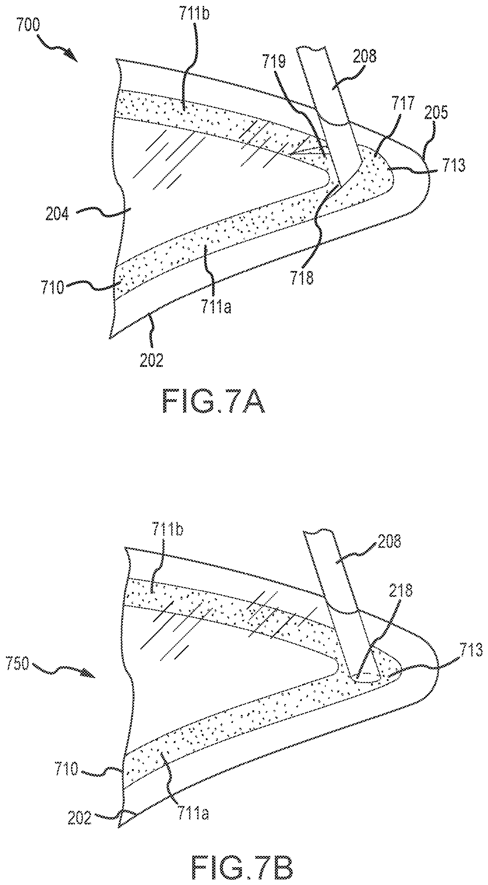

**FIG. 7A** is a front isometric view of the distal end region of fluid collection device 700. Although only the distal end region of the fluid collection device 700 is shown, unless otherwise noted, the fluid collection device 700 may include any aspect of the fluid collection device 200, such as the fluid impermeable barrier 202, the opening (not shown), and the aperture 212. The fluid collection device 700 also includes a fluid permeable body 710. Unless otherwise noted, the fluid permeable body 710 may include any aspect of the fluid permeable body 210, such as the materials, continuity, and uniformity described in relation to the fluid permeable body 210. The fluid permeable body 710 may be V-shaped and include two (or more) arms 711a, 711b that meet at a distal portion 713 of the fluid permeable body 710 proximate to the distal end region 205 of the fluid impermeable barrier 202. The arms 711a, 711b extend from two different portions of the chamber to a common area at the distal portion 713, thereby allowing the tube 208 to draw urine from more than one direction within the chamber 204. The two arms 711a, 711b may extend from an integral distal portion 713 of the fluid permeable body 710. In some embodiments, the two arms 711a, 711b may be two separate elements positioned proximate or adjacent to another to form the distal portion 713 of the fluid permeable body 710.

The distal portion 713 of the fluid permeable body 710 may include a crescent-shaped protrusion 717 positioned proximate to the distal end region 205 of the fluid impermeable barrier 202 and a base 719. The protrusion may include other shapes, and may be configured to include a pocket or tunnel. The base 719 and the protrusion 717 may include any aspect of the fluid permeable body 710, including the fluid permeable materials. A pocket 718 may be defined between the protrusion 717 and the base 719 of the distal portion 713. The pocket 718 may be sized to hold a portion of the tube 208 therein, including the end of the tube 208 and the tube opening on the tube 208. In some embodiments, such as the urine collection device 750 shown in **FIG. 7B****,** the protrusion 717 is absent, and the tube opening 218 may be positioned on the distal portion 713 of the fluid permeable barrier 710, such as on top or imbedded within the fluid permeable body 710.

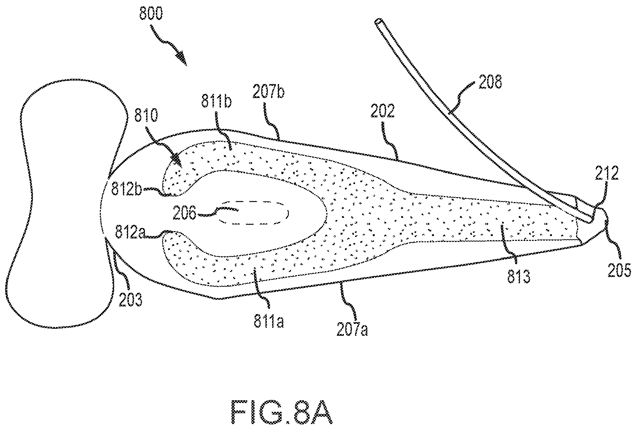

**FIG. 8A** is a front isometric view of a urine collection device 800. Unless otherwise noted, the urine collection device 800 may include any aspect of the urine collection devices 200, 300, 400, 500, 600, 650, 700, 750. The urine collection device 800 may include a fluid impermeable barrier 202 defining a chamber 204, an aperture 212, and an opening 206, and having a proximal end region 203, and a distal end region 205. The fluid impermeable barrier 202 may narrow from the proximal end region 203 to the distal end region 205 and may include the first edge 207a and the second edge 207b extending at least partially between the distal end region 205 and the proximal end region 203.

The urine collection device 800 includes a fluid permeable body 810 having a distal portion 813 and two or more arms 811a, 811b extending from the distal portion 813. Unless otherwise noted, the fluid permeable body 810 may include any aspect of the fluid permeable body 210, such as materials, dimensions, uniformity, and continuity. For example, each of the arms 811a, 811b and the distal portion 813 may include an outer fluid permeable wicking material and a fluid permeable porous fiber structure. The fluid permeable body of the arms 811a, 811b and the distal portion 813 may be substantially uniform and continuous from terminating ends 812a, 812b of the arms 811a, 811b to the distal portion 813 of the fluid permeable body 810.

In some embodiments, the fluid permeable body 810 includes a generally wishbone-shaped configuration. The distal portion 813 of the fluid permeable body may extend from proximate to the distal end region 205 of the fluid impermeable barrier 202 at least about one-half, at least about two-thirds, at least about three-quarters, at least about seven-eighths, or substantially all of the distance from the distal end region 205 of the fluid impermeable barrier 202 to the opening 206. In the urine collection device 800, a gap or space is positioned between the opening and the distal portion 813 of the fluid permeable body 810. In some embodiments, the opening 206 may substantially abut the distal portion 813 of the fluid permeable body 810. The distal portion 813 may include a substantially planar surface from the opening 206 or proximate to the opening 206 to the distal end region 205 or proximate to the distal end region 205 of the fluid impermeable barrier 202.

The fluid permeable body 810 also includes two or more arms 811a, 811b that extend from the distal portion 813 at least partially around the opening 206. For example, the first arm 811a may extend or wrap at least partially around a first side of the opening 206 and the second arm 811b may extend or wrap at least partially around a second side of the opening 206. Each of the arms 811a, 811b include a terminating end 812a, 812b. The terminating ends 812a, 812b may be substantially distal to the distal portion 813, with the opening 206 positioned between the terminating ends 812a, 812b and the distal portion 813. In some embodiments, a space or gap may be positioned between the terminating ends 812a, 812b. In some embodiments, the terminating ends 812a, 812b may be adjacent to one another and/or abut one another.

The distal portion 813 of the fluid permeable body 810 may be spaced between the edges 207a, 207b of the fluid impermeable barrier 202. In some embodiments, the distal portion 813 of the fluid permeable body 810 may abut or interface at least a portion of the edges 207a, 207b. The arms 811a, 811b of the fluid permeable body 810 may be spaced from the edges 207a, 207b, respectively. In some embodiments, the arms 811a, 811b of the fluid permeable body may abut or interface at least a portion of the edges 207a, 207b.

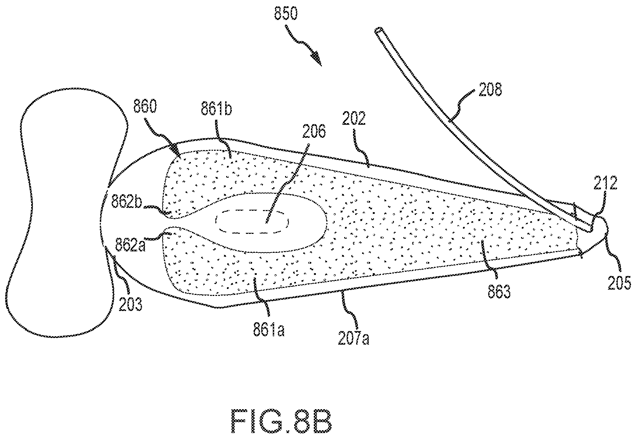

**FIG. 8B** is a front isometric view of a urine collection device 850. Unless otherwise noted, the urine collection device 850 may include any aspect of the urine collection devices 200, 300, 400, 500, 600, 650, 700, 750, 800. The urine collection device 850 may include a fluid impermeable barrier 202 defining a chamber 204, an aperture 212, and an opening 206, and having a proximal end region 203 and a distal end region 205. The fluid impermeable barrier 202 may narrow from the proximal end region 203 to the distal end region 205 and may include the first edge 207a and the second edge 207b extending at least partially between the distal end region 205 and the proximal end region 203.

The urine collection device 850 includes a fluid permeable body 860 having a distal portion 863 and arms 861a, 861b extending from the distal portion 863. Unless otherwise noted, the fluid permeable body 860 may include any aspect of the fluid permeable body 210, such as materials, dimensions, uniformity, and continuity. For example, each of the arms 861a, 861b and the distal portion 863 may include an outer fluid permeable wicking material and a fluid permeable porous fiber structure. The fluid permeable body of the arms 861a, 861b and the distal portion 863 may be substantially uniform and continuous from terminating ends 862a, 862b of the arms 861a, 861b to the distal portion 863 of the fluid permeable body 860.

In some embodiments, the fluid permeable body 860 includes a shape generally complementary to the shape of the fluid permeable barrier 202 and/or the chamber 204 defined by the fluid impermeable barrier 202. The distal portion 863 of the fluid permeable body may extend from proximate to the distal end region 205 of the fluid impermeable barrier 202 at least about one-half, at least about two-thirds, at least about three-quarters, at least about seven-eighths, or substantially all of the distance from the distal end region 205 of the fluid impermeable barrier 202 to the opening 206. In the urine collection device 850, a gap or space is positioned between the opening and the distal portion 863 of the fluid permeable body 860. In some embodiments, the opening 206 may substantially abut the distal portion 863 of the fluid permeable body 860. The distal portion 863 may include a substantially planar surface from the opening 206 or proximate to the opening 206 to the distal end region 205 or proximate to the distal end region 205 of the fluid impermeable barrier 202.

The fluid permeable body 860 also includes arms 861a, 861b that extend from the distal portion 863 at least partially around the opening 206. For example, the first arm 861a may extend or wrap at least partially around a first side of the opening 206 and the second arm 861b may extend or wrap at least partially around a second side of the opening 206. Each of the arms 861a, 861b include a terminating end 862a, 862b. The terminating ends 862a, 862b may be substantially distal to the distal portion 863, with the opening 206 positioned between the terminating ends 862a, 862b and the distal portion 863. In some embodiments, a space or gap may be positioned between the terminating ends 862a, 862b. In some embodiments, the terminating ends 862a, 862b may be adjacent to one another and/or abut one another.

The distal portion 863 of the fluid permeable body 860 may be spaced between the edges 207a, 207b of the fluid impermeable barrier 202. In some embodiments, the distal portion 863 of the fluid permeable body 860 may abut or interface at least a portion of the edges 207a, 207b. In some embodiments, the distal portion 863 may abut or interface the edges 207a, 207b along an entire length of the distal portion 863 such that the distal portion 863 substantially fills a distance between the edges 207a, 207b in the chamber 204. The arms 811a, 811b of the fluid permeable body 810 may be spaced from the edges 207a, 207b, respectively. In some embodiments, the arms 811a, 811b of the fluid permeable body 810 may abut or interface at least a portion of the edges 207a, 207b. In some embodiments, the arms 811a, 811b may abut or interface the edges 207a, 207b along an entire length of the arms 811a, 811b.

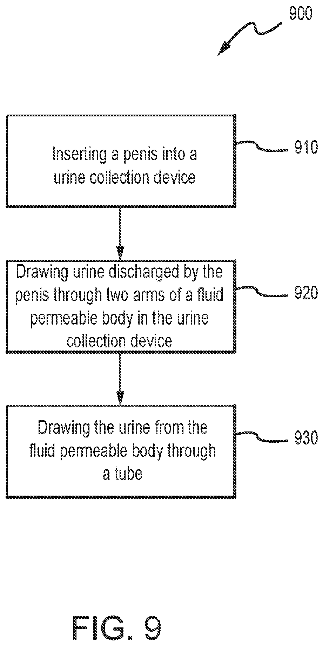

**FIG. 9** is a flow diagram of a method 900 for collecting fluid from a user, according to an embodiment. The method 900 includes an act 910 of inserting a penis into a urine collection device. The method 900 includes an act 920 of drawing urine discharged by the penis through two arms of a fluid permeable body in the urine collection device. The method includes an act 930 of drawing the urine from the fluid permeable body through a tube.

Any of the fluid (including urine) collection devices described herein may be used in the method 900. In some embodiments, the act 910 includes inserting a penis through an opening in a fluid impermeable barrier of a urine collection device into a chamber of the urine collection device at least partially defined by the fluid impermeable barrier.

The act 920 may include drawing urine discharged by the penis in the chamber through two arms of a fluid permeable body to a distal portion of the fluid permeable body at least partially positioned proximate to a distal end region of the fluid impermeable barrier, the two arms being spaced from one another and extending from the distal portion of the fluid permeable body. In some embodiments, the act 920 of drawing the urine from the distal portion of the fluid permeable body through the tube may include drawing the urine from the distal portion of the fluid permeable body through a tube opening in the tube that is covered by the distal portion of the fluid permeable body. In some embodiments, the act 920 of drawing the urine from the distal portion of the fluid permeable body through the tube may include drawing the urine from the distal portion of the fluid permeable body through a tube opening of the tube positioned in a pocket defined between a protrusion on the fluid permeable body and the distal portion of the fluid permeable body.

In some embodiments, the act 920 of drawing urine discharged by the penis in the chamber through two arms of a fluid permeable body to a distal portion of the fluid permeable body may include drawing the urine through an outer fluid permeable wicking material and a fluid permeable porous fiber structure on each of the two arms to the distal portion of the fluid permeable body. Drawing the urine through an outer fluid permeable wicking material and a fluid permeable porous fiber structure on each of the two arms to the distal portion of the fluid permeable body may include drawing the urine through the outer fluid permeable wicking material and the fluid permeable porous fiber structure on each of the two arms to a fluid permeable porous fiber structure of the distal portion of the fluid permeable body that is continuous with the fluid permeable porous fiber structure of the two arms.

In some embodiments, the act 920 of drawing urine discharged by the penis in the chamber through two arms of a fluid permeable body to a distal portion of the fluid permeable body may include drawing the urine discharged by the penis in the chamber through the two arms of the fluid permeable body positioned at opposing edges of the chamber as the chamber narrows to a tip proximate the distal portion of the fluid permeable body. In some embodiments, the act 920 of drawing urine discharged by the penis in the chamber through two arms of a fluid permeable body to a distal portion of the fluid permeable body may include drawing urine discharged by the penis in the chamber through the two arms of the fluid permeable body to the distal portion of the fluid permeable body being at least covered by a fluid impermeable membrane that holds the urine in the distal portion of the fluid permeable body.

The method 900 also may include an act of drawing the urine discharged by the penis in the chamber through one or more additional arms of the fluid permeable body to the distal portion of the fluid permeable body, the one or more additional arms being positioned within the chamber between the first arm and the second arm. The method 900 also may include an act of positioning the urine collection device substantially horizontal on the user. The act 920 of drawing the urine from the distal portion of the fluid permeable body through the tube, then, may include drawing urine through a tube opening at a beveled end of the tube positioned on the distal portion of the fluid permeable body, the tube angling relative to distal portion of the fluid permeable body from the beveled end through an aperture in the fluid impermeable barrier and out of the chamber.

The acts of the method 900 described above are for illustrative purposes. For example, the acts of the method 900 can be performed in different orders, split into multiple acts, modified, supplemented, or combined. In an embodiment, one or more of the act of the method 900 can be omitted from the method 900. Any of the acts of the method 900 can include using any of the urine collection systems disclosed herein.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

## Claims

1. A urine collection device, comprising:
a fluid impermeable barrier (202) at least partially defining an opening (206) sized to receive at least a portion of a penis and a chamber (204) within the fluid collection device in fluid communication with the opening, the fluid impermeable barrier having a bottom layer, a top layer opposite to the bottom layer, a proximal end region and a distal end region, wherein the opening (206) is on the bottom layer,
a fluid permeable body (210, 310, 410, 510, 610, 660, 710, 810, 860) positioned within the chamber and including a distal portion (213, 313, 413, 513, 613, 663, 713, 813, 863) positioned at least partially in or proximate to the distal end region of the fluid impermeable barrier, a first arm (211a, 311a, 411a, 511a, 611a, 661a, 711a, 811a, 861a) extending from the distal portion of the fluid permeable body towards the proximal end region of the fluid impermeable barrier, and a second arm (211b, 311b, 411b, 511b, 611b, 661b, 711b, 811b, 861b) spaced from the first arm and extending from the distal portion of the fluid permeable body towards the proximal end region of the fluid impermeable barrier; and
a tube (208) extending into the chamber and having an end positioned proximate to the distal end region of the fluid impermeable barrier, wherein the tube includes a tube opening proximate to the end of the tube and at least a portion of the fluid permeable body is positioned between the fluid impermeable barrier and the end of the tube, **characterised in that** the opening is positioned between the proximal end region and the distal end region of the bottom portion of the fluid impermeable barrier.

2. The urine collection device of claim 1, wherein the fluid permeable body includes a continuous planar surface on the first arm, the second arm, and the distal portion.

3. The urine collection device of any of claims 1-2, wherein at least the first arm and the second arm of the fluid permeable body include a fluid permeable porous fiber structure (521) and an outer fluid permeable wicking material (523), and the distal portion of the fluid permeable body includes at least the fluid permeable porous fiber structure.

4. The urine collection device of any of claims 1-3, wherein the distal portion of the fluid permeable body includes the fluid permeable porous fibers and the outer fluid permeable wicking material, wherein the fluid permeable porous fiber structure includes a uniform thickness in at least the distal portion of the fluid permeable body; and the fluid permeable porous fiber structure includes a uniform thickness in the first arm, the second arm, and the distal portion of the fluid permeable body.

5. The urine collection device of any of claims 1-4, wherein the distal portion of the fluid permeable body extends from the distal end region of the fluid impermeable barrier to the opening and includes a continuous planar surface from the opening to the distal end region of the fluid impermeable barrier; wherein the first arm and the second arm extend at least partially around the opening, wherein the first arm includes a first terminal end and the second arm includes a second terminal end adjacent to the first terminal end, the opening being positioned between the distal portion and the first terminal end and the second terminal end.

6. The urine collection device of claim 5, wherein:
the fluid impermeable barrier narrows from the proximal end region to a tip at the distal end region and includes a first edge (207a) and a second edge (207b) extending at least partially between the distal end region and the proximal end region;
the distal portion of the fluid permeable body is positioned at least proximate to at least a portion of the first edge and at least a portion of the second edge;
the first arm is positioned at least proximate to at least a portion of the first edge such that the fluid permeable body extends continuously along the first edge from the distal end region of the fluid impermeable barrier beyond at least a portion of the opening; and
the second arm is positioned at least proximate to at least a portion of the second edge such that the fluid permeable body extends continuously along the second edge from the distal end region of the fluid impermeable barrier beyond at least a portion of the opening.

7. The urine collection device of claim 5, wherein:
the fluid impermeable barrier narrows from the proximal end region to a tip at the distal end region and includes a first edge (207a) and a second edge (207b) extending at least partially between the distal end region and the proximal end region;
the distal portion of the fluid permeable body is spaced between the first edge and the second edge;
the first arm curls around a portion of the opening and is spaced from the first edge; and
the second arm curls around a portion of the opening and is spaced from the second edge.

8. The urine collection device of any of claims 1-4, wherein the distal portion of the fluid permeable body extends less than one-third of a distance between the terminal end of the chamber and the opening, wherein:
the fluid impermeable barrier narrows from the proximal end region to a tip at the distal end region and includes a first edge (207a) and a second edge (207b) extending at least partially between the distal end region and the proximal end region;
the first arm is positioned at least proximate to at least a portion of the first edge; and
the second arm is positioned at least proximate to at least a portion of the second edge, the device further comprising one or more additional arms extending from the distal portion of the fluid permeable body, the one or more additional arms being positioned within the chamber between the first arm and the second arm, wherein each of the one or more additional arms include a fluid permeable porous fiber structure and an outer fluid permeable wicking material, and wherein each of the one or more additional arms is planar.

9. The urine collection device of claim 8, wherein the first arm, the second arm, and the distal portion of the fluid permeable body form a V-shaped configuration.

10. The urine collection device of claim 8, wherein the first arm, the second arm, and the distal portion of the fluid permeable body form a U-shaped configuration.

11. The urine collection device of claim 8, wherein the first arm, the second arm, and the distal portion of the fluid permeable body form a Y-shaped configuration.

12. The urine collection device of any of claims 1-11, wherein:
the fluid impermeable barrier includes a top portion defining an aperture with the tube extending therethrough and a bottom portion defining the opening, the urine collection device being configured to be horizontal during use with the fluid permeable body and the bottom portion of the fluid impermeable barrier being horizontal; and
the end of the tube positioned in the chamber includes a beveled end with the tube opening positioned at the beveled end and the tube angles relative to distal portion of the fluid permeable body from the beveled end towards the aperture and out of the chamber.

13. The urine collection device of any of claims 1-12, wherein the distal portion of fluid permeable body covers the tube opening.

14. The urine collection device of any of claims 1-13, wherein the fluid permeable body includes a protrusion on the distal portion of the fluid permeable body proximate to the distal end region of the fluid impermeable barrier and a pocket (318) defined between the protrusion and the distal portion of the fluid permeable body, the tube opening and the end of the tube being positioned within the pocket.

15. A method of collecting urine, the method comprising:
inserting a penis through an opening (206) in a bottom layer of a fluid impermeable barrier (202) of a urine collection device ,according to any one of claims 1-14, into a chamber (204) of the urine collection device at least partially defined by the fluid impermeable barrier, wherein the opening (206) is on the bottom layer, wherein the opening is positioned between the proximal end region and the distal end region of the bottom portion of the fluid impermeable barrier;
drawing urine discharged by the penis in the chamber through two arms (211a,b; 311a,b; 411a,b; 511a,b; 611a,b; 661a,b; 711a,b; 811a,b; 861a,b) of a fluid permeable body (210, 310, 410, 510, 610, 660, 710, 810, 860) to a distal portion (213, 313, 413, 513, 613, 663, 713, 813, 863) of the fluid permeable body at least partially positioned proximate to a distal end region of the fluid impermeable barrier, the two arms being spaced from one another and extending from the distal portion of the fluid permeable body; and
drawing the urine from the distal portion of the fluid permeable body through a tube (208).

## Patentansprüche

1. Urinsammelvorrichtung, umfassend:
eine flüssigkeitsundurchlässige Barriere (202), die mindestens teilweise eine Öffnung (206) definiert, die dazu bemessen ist, zumindest einen Abschnitt eines Penis aufzunehmen, und eine Kammer (204) innerhalb der Flüssigkeitssammelvorrichtung, die in Fluidverbindung mit der Öffnung steht, wobei die flüssigkeitsundurchlässige Barriere einen Bodenabschnitt, einen oberen Abschnitt gegenüber dem Bodenabschnitt, einen proximalen Endbereich und einen distalen Endbereich aufweist, wobei die Öffnung (206) an dem Bodenabschnitt angeordnet ist,
einen flüssigkeitsdurchlässigen Körper (210, 310, 410, 510, 610, 660, 710, 810, 860), der innerhalb der Kammer positioniert ist und einen distalen Abschnitt (213, 313, 413, 513, 613, 663, 713, 813, 863) einschließt, der zumindest teilweise in oder in der Nähe des distalen Endbereichs der flüssigkeitsundurchlässigen Barriere positioniert ist, einen ersten Arm (211a, 311a, 411a, 511a, 611a, 661a, 711a, 811a, 861a), der sich von dem distalen Abschnitt des flüssigkeitsdurchlässigen Körpers in Richtung des proximalen Endbereichs der flüssigkeitsundurchlässigen Barriere erstreckt, und einen zweiten Arm (211b, 311b, 411b, 511b, 611b, 661b, 711b, 811b, 861b), der von dem ersten Arm beabstandet ist und sich von dem distalen Abschnitt des flüssigkeitsdurchlässigen Körpers in Richtung des proximalen Endbereichs der flüssigkeitsundurchlässigen Barriere erstreckt; und
einen Schlauch (208), der sich in die Kammer hinein erstreckt und ein Ende aufweist, das in der Nähe des distalen Endbereichs der flüssigkeitsundurchlässigen Barriere positioniert ist, wobei der Schlauch eine Schlauchöffnung in der Nähe des Endes des Schlauchs einschließt und mindestens ein Abschnitt des flüssigkeitsdurchlässigen Körpers zwischen der flüssigkeitsundurchlässigen Barriere und dem Ende des Schlauchs positioniert ist, **dadurch gekennzeichnet, dass** die Öffnung zwischen dem proximalen Endbereich und dem distalen Endbereich des Bodenabschnitts der flüssigkeitsundurchlässigen Barriere positioniert ist.

2. Urinsammelvorrichtung nach Anspruch 1, wobei der flüssigkeitsdurchlässige Körper eine durchgehende ebene Oberfläche auf dem ersten Arm, dem zweiten Arm und dem distalen Abschnitt einschließt.

3. Urinsammelvorrichtung nach einem der Ansprüche 1-2, wobei zumindest der erste Arm und der zweite Arm des flüssigkeitsdurchlässigen Körpers eine flüssigkeitsdurchlässige poröse Faserstruktur (521) und ein äußeres flüssigkeitsdurchlässiges Dochtmaterial (523) einschließen, und wobei der distale Abschnitt des flüssigkeitsdurchlässigen Körpers zumindest die flüssigkeitsdurchlässige poröse Faserstruktur einschließt.

4. Urinsammelvorrichtung nach einem der Ansprüche 1-3, wobei der distale Abschnitt des flüssigkeitsdurchlässigen Körpers die flüssigkeitsdurchlässigen porösen Fasern und das äußere flüssigkeitsdurchlässige Dochtmaterial einschließt, wobei die flüssigkeitsdurchlässige poröse Faserstruktur eine gleichmäßige Dicke in zumindest dem distalen Abschnitt des flüssigkeitsdurchlässigen Körpers einschließt; und wobei die flüssigkeitsdurchlässige poröse Faserstruktur eine gleichmäßige Dicke in dem ersten Arm, dem zweiten Arm und dem distalen Abschnitt des flüssigkeitsdurchlässigen Körpers einschließt.

5. Urinsammelvorrichtung nach einem der Ansprüche 1-4, wobei sich der distale Abschnitt des flüssigkeitsdurchlässigen Körpers von dem distalen Endbereich der flüssigkeitsundurchlässigen Barriere bis zu der Öffnung erstreckt und eine durchgehende ebene Oberfläche von der Öffnung bis zu dem distalen Endbereich der flüssigkeitsundurchlässigen Barriere einschließt; wobei sich der erste Arm und der zweite Arm mindestens teilweise um die Öffnung herum erstrecken, wobei der erste Arm einen ersten Endabschnitt einschließt und der zweite Arm einen zweiten Endabschnitt einschließt, der an den ersten Endabschnitt angrenzt, wobei die Öffnung zwischen dem distalen Abschnitt und dem ersten Endabschnitt und dem zweiten Endabschnitt positioniert ist.

6. Urinsammelvorrichtung nach Anspruch 5, wobei:
die flüssigkeitsundurchlässige Barriere sich von dem proximalen Endbereich zu einer Spitze an dem distalen Endbereich verjüngt und einen ersten Rand (207a) und einen zweiten Rand (207b) einschließt, die sich mindestens teilweise zwischen dem distalen Endbereich und dem proximalen Endbereich erstrecken;
der distale Abschnitt des flüssigkeitsdurchlässigen Körpers zumindest in der Nähe von zumindest einem Abschnitt des ersten Randes und zumindest einem Abschnitt des zweiten Randes positioniert ist;
der erste Arm zumindest in der Nähe von zumindest einem Abschnitt des ersten Randes positioniert ist, so dass sich der flüssigkeitsdurchlässige Körper durchgehend entlang des ersten Randes von dem distalen Endbereich der flüssigkeitsundurchlässigen Barriere über zumindest einen Abschnitt der Öffnung hinaus erstreckt; und
der zweite Arm zumindest in der Nähe von zumindest einem Abschnitt des zweiten Randes positioniert ist, so dass sich der flüssigkeitsdurchlässige Körper durchgehend entlang des zweiten Randes von dem distalen Endbereich der flüssigkeitsundurchlässigen Barriere über zumindest einen Abschnitt der Öffnung hinaus erstreckt.

7. Urinsammelvorrichtung nach Anspruch 5, wobei:
die flüssigkeitsundurchlässige Barriere sich von dem proximalen Endbereich zu einer Spitze an dem distalen Endbereich verjüngt und einen ersten Rand (207a) und einen zweiten Rand (207b) einschließt, die sich mindestens teilweise zwischen dem distalen Endbereich und dem proximalen Endbereich erstrecken;
der distale Abschnitt des flüssigkeitsdurchlässigen Körpers zwischen dem ersten Rand und dem zweiten Rand beabstandet ist;
der erste Arm sich um einen Abschnitt der Öffnung herum krümmt und von dem ersten Rand beabstandet ist; und
der zweite Arm sich um einen Abschnitt der Öffnung herum krümmt und von dem zweiten Rand beabstandet ist.

8. Urinsammelvorrichtung nach einem der Ansprüche 1-4, wobei sich der distale Abschnitt des flüssigkeitsdurchlässigen Körpers weniger als ein Drittel einer Strecke zwischen dem Endabschnitt der Kammer und der Öffnung erstreckt, wobei:
die flüssigkeitsundurchlässige Barriere sich von dem proximalen Endbereich zu einer Spitze an dem distalen Endbereich verjüngt und einen ersten Rand (207a) und einen zweiten Rand (207b) einschließt, die sich mindestens teilweise zwischen dem distalen Endbereich und dem proximalen Endbereich erstrecken;
der erste Arm zumindest in der Nähe von zumindest einem Abschnitt des ersten Randes positioniert ist; und
der zweite Arm zumindest in der Nähe von zumindest einem Abschnitt des zweiten Randes positioniert ist, wobei die Vorrichtung weiter einen oder mehrere zusätzliche Arme umfasst, die sich von dem distalen Abschnitt des flüssigkeitsdurchlässigen Körpers erstrecken, wobei die einen oder mehreren zusätzlichen Arme innerhalb der Kammer zwischen dem ersten Arm und dem zweiten Arm positioniert sind, wobei jeder des einen oder der mehreren zusätzlichen Arme eine flüssigkeitsdurchlässige poröse Faserstruktur und ein äußeres flüssigkeitsdurchlässiges Dochtmaterial einschließt, und wobei jeder der einen oder mehreren zusätzlichen Arme planar ist.

9. Urinsammelvorrichtung nach Anspruch 8, wobei der erste Arm, der zweite Arm und der distale Abschnitt des flüssigkeitsdurchlässigen Körpers eine V-förmige Konfiguration bilden.

10. Urinsammelvorrichtung nach Anspruch 8, wobei der erste Arm, der zweite Arm und der distale Abschnitt des flüssigkeitsdurchlässigen Körpers eine U-förmige Konfiguration bilden.

11. Urinsammelvorrichtung nach Anspruch 8, wobei der erste Arm, der zweite Arm und der distale Abschnitt des flüssigkeitsdurchlässigen Körpers eine Y-förmige Konfiguration bilden.

12. Urinsammelvorrichtung nach einem der Ansprüche 1-11, wobei:
die flüssigkeitsundurchlässige Barriere einen oberen Abschnitt, der einen Durchlass definiert, wobei sich der Schlauch dadurch hindurch erstreckt, und einen Bodenabschnitt einschließt, der die Öffnung definiert, wobei die Urinsammelvorrichtung dazu ausgebildet ist, während des Gebrauchs horizontal zu sein, wobei der flüssigkeitsdurchlässige Körper und der Bodenabschnitt der flüssigkeitsundurchlässigen Barriere horizontal sind; und
das in der Kammer positionierte Ende des Schlauches ein abgeschrägtes Ende einschließt, wobei die Schlauchöffnung an dem abgeschrägten Ende positioniert ist und der Schlauch relativ zu dem distalen Abschnitt des flüssigkeitsdurchlässigen Körpers von dem abgeschrägten Ende in Richtung des Durchlasses und aus der Kammer heraus abgewinkelt ist.

13. Urinsammelvorrichtung nach einem der Ansprüche 1-12, wobei der distale Abschnitt des flüssigkeitsdurchlässigen Körpers die Schlauchöffnung abdeckt.

14. Urinsammelvorrichtung nach einem der Ansprüche 1-13, wobei der flüssigkeitsdurchlässige Körper einen Vorsprung auf dem distalen Abschnitt des flüssigkeitsdurchlässigen Körpers in der Nähe des distalen Endbereichs der flüssigkeitsundurchlässigen Barriere einschließt und eine Tasche (318) zwischen dem Vorsprung und dem distalen Abschnitt des flüssigkeitsdurchlässigen Körpers definiert ist, wobei die Schlauchöffnung und das Ende des Schlauches innerhalb der Tasche positioniert sind.

15. Verfahren zum Sammeln von Urin, wobei das Verfahren Folgendes umfasst:
Einführen eines Penis durch eine Öffnung (206) in einem Bodenabschnitt einer flüssigkeitsundurchlässigen Barriere (202) einer Urinsammelvorrichtung, nach einem der Ansprüche 1-14, in eine Kammer (204) der Urinsammelvorrichtung, die mindestens teilweise durch die flüssigkeitsundurchlässige Barriere definiert ist, wobei die Öffnung (206) an dem Bodenabschnitt angeordnet ist, wobei die Öffnung zwischen dem proximalen Endbereich und dem distalen Endbereich des Bodenabschnitts der flüssigkeitsundurchlässigen Barriere positioniert ist;
Abziehen von durch den Penis in der Kammer abgegebenem Urin durch zwei Arme (211a,b; 311a,b; 411a,b; 511a,b; 611a,b; 661a,b; 711a,b; 811a,b; 861a,b) eines flüssigkeitsdurchlässigen Körpers (210, 310, 410, 510, 610, 660, 710, 810, 860) zu einem distalen Abschnitt (213, 313, 413, 513, 613, 663, 713, 813, 863) des flüssigkeitsdurchlässigen Körpers, der zumindest teilweise in der Nähe eines distalen Endbereichs der flüssigkeitsundurchlässigen Barriere positioniert ist, wobei die zwei Arme voneinander beabstandet sind und sich von dem distalen Abschnitt des flüssigkeitsdurchlässigen Körpers erstrecken; und
Abziehen des Urins von dem distalen Abschnitt des flüssigkeitsdurchlässigen Körpers durch einen Schlauch (208).

## Revendications

1. Dispositif de collecte d'urine, comprenant :
une barrière (202) imperméable aux fluides définissant au moins partiellement une ouverture (206) dimensionnée pour recevoir au moins une partie d'un pénis et une chambre (204) à l'intérieur du dispositif de collecte de fluide en communication fluidique avec l'ouverture, la barrière imperméable aux fluides présentant une couche inférieure, une couche supérieure opposée à la couche inférieure, une région d'extrémité proximale et une région d'extrémité distale, dans lequel l'ouverture (206) se trouve sur la couche inférieure,
un corps (210, 310, 410, 510, 610, 660, 710, 810, 860) perméable aux fluides positionné à l'intérieur de la chambre et incluant une partie distale (213, 313, 413, 513, 613, 663, 713, 813, 863) positionnée au moins partiellement dans ou à proximité de la région d'extrémité distale de la barrière imperméable aux fluides,
un premier bras (211a, 311a, 411a, 511a, 611a, 661a, 711a, 811a, 861a) s'étendant à partir de la partie distale du corps perméable aux fluides vers la région d'extrémité proximale de la barrière imperméable aux fluides, et un deuxième bras (211b, 311b, 411b, 511b, 611b, 661b, 711b, 811b, 861b) espacé du premier bras et s'étendant à partir de la partie distale du corps perméable aux fluides vers la région d'extrémité proximale de la barrière imperméable aux fluides ; et
un tube (208) s'étendant dans la chambre et présentant une extrémité positionnée à proximité de la région d'extrémité distale de la barrière imperméable aux fluides,
dans lequel le tube inclut une ouverture de tube à proximité de l'extrémité du tube et au moins une partie du corps perméable aux fluides est positionnée entre la barrière imperméable aux fluides et l'extrémité du tube, **caractérisé en ce que** l'ouverture est positionnée entre la région d'extrémité proximale et la région d'extrémité distale de la partie inférieure de la barrière imperméable aux fluides.

2. Dispositif de collecte d'urine selon la revendication 1, dans lequel le corps perméable aux fluides inclut une surface plane continue sur le premier bras, le deuxième bras, et la partie distale.

3. Dispositif de collecte d'urine selon l'une quelconque de la revendication 1 et de la revendication 2, dans lequel au moins le premier bras et le deuxième bras du corps perméable aux fluides incluent une structure (521) fibreuse poreuse perméable aux fluides et un matériau (523) à effet de mèche perméable aux fluides extérieur, et la partie distale du corps perméable aux fluides inclut au moins la structure fibreuse poreuse perméable aux fluides.

4. Dispositif de collecte d'urine selon l'une quelconque des revendications 1 à 3, dans lequel la partie distale du corps perméable aux fluides inclut les fibres poreuses perméables aux fluides et le matériau à effet de mèche perméable aux fluides extérieur, dans lequel la structure fibreuse poreuse perméable aux fluides inclut une épaisseur uniforme dans au moins la partie distale du corps perméable aux fluides ; et la structure fibreuse poreuse perméable aux fluides inclut une épaisseur uniforme dans le premier bras, le deuxième bras, et la partie distale du corps perméable aux fluides.

5. Dispositif de collecte d'urine selon l'une quelconque des revendications 1 à 4, dans lequel la partie distale du corps perméable aux fluides s'étend de la région d'extrémité distale de la barrière imperméable aux fluides à l'ouverture et inclut une surface plane continue de l'ouverture à la région d'extrémité distale de la barrière imperméable aux fluides ; dans lequel le premier bras et le deuxième bras s'étendent au moins partiellement autour de l'ouverture, dans lequel le premier bras inclut une première extrémité terminale et le deuxième bras inclut une deuxième extrémité terminale adjacente à la première extrémité terminale, l'ouverture étant positionnée entre la partie distale et la première extrémité terminale et la deuxième extrémité terminale.

6. Dispositif de collecte d'urine selon la revendication 5, dans lequel :
la barrière imperméable aux fluides se rétrécit à partir de la région d'extrémité proximale jusqu'à une pointe au niveau de la région d'extrémité distale et inclut un premier bord (207a) et un deuxième bord (207b) s'étendant au moins partiellement entre la région d'extrémité distale et la région d'extrémité proximale ;
la partie distale du corps perméable aux fluides est positionnée au moins à proximité d'au moins une partie du premier bord et d'au moins une partie du deuxième bord ;
le premier bras est positionné au moins à proximité d'au moins une partie du premier bord de sorte que le corps perméable aux fluides s'étende de façon continue le long du premier bord à partir de la région d'extrémité distale de la barrière imperméable aux fluides au-delà d'au moins une partie de l'ouverture ; et
le deuxième bras est positionné au moins à proximité d'au moins une partie du deuxième bord de sorte que le corps perméable aux fluides s'étende de façon continue le long du deuxième bord à partir de la région d'extrémité distale de la barrière imperméable aux fluides au-delà d'au moins une partie de l'ouverture.

7. Dispositif de collecte d'urine selon la revendication 5, dans lequel :
la barrière imperméable aux fluides se rétrécit à partir de la région d'extrémité proximale jusqu'à une pointe au niveau de la région d'extrémité distale et inclut un premier bord (207a) et un deuxième bord (207b) s'étendant au moins partiellement entre la région d'extrémité distale et la région d'extrémité proximale ;
la partie distale du corps perméable aux fluides est espacée entre le premier bord et le deuxième bord ;
le premier bras s'enroule autour d'une partie de l'ouverture et est espacé du premier bord ; et
le deuxième bras s'enroule autour d'une partie de l'ouverture et est espacé du deuxième bord.

8. Dispositif de collecte d'urine selon l'une quelconque des revendications 1 à 4, dans lequel la partie distale du corps perméable aux fluides s'étend sur moins d'un tiers d'une distance entre l'extrémité terminale de la chambre et l'ouverture, dans lequel :
la barrière imperméable aux fluides se rétrécit à partir de la région d'extrémité proximale jusqu'à une pointe au niveau de la région d'extrémité distale et inclut un premier bord (207a) et un deuxième bord (207b) s'étendant au moins partiellement entre la région d'extrémité distale et la région d'extrémité proximale ;
le premier bras est positionné au moins à proximité d'au moins une partie du premier bord ; et
le deuxième bras est positionné au moins à proximité d'au moins une partie du deuxième bord, le dispositif comprenant en outre un ou plusieurs bras supplémentaires s'étendant à partir de la partie distale du corps perméable aux fluides, le un ou les plusieurs bras supplémentaires étant positionnés à l'intérieur de la chambre entre le premier bras et le deuxième bras, dans lequel chacun du un ou des plusieurs bras supplémentaires incluent une structure fibreuse poreuse perméable aux fluides et un matériau à effet de mèche perméable aux fluides extérieur, et dans lequel chacun du un ou des plusieurs bras supplémentaires est plan.

9. Dispositif de collecte d'urine selon la revendication 8, dans lequel le premier bras, le deuxième bras, et la partie distale du corps perméable aux fluides forment une configuration en forme de V.

10. Dispositif de collecte d'urine selon la revendication 8, dans lequel le premier bras, le deuxième bras, et la partie distale du corps perméable aux fluides forment une configuration en forme de U.

11. Dispositif de collecte d'urine selon la revendication 8, dans lequel le premier bras, le deuxième bras, et la partie distale du corps perméable aux fluides forment une configuration en forme de Y.

12. Dispositif de collecte d'urine selon l'une quelconque des revendications 1 à 11, dans lequel :
la barrière imperméable aux fluides inclut une partie supérieure définissant un orifice avec le tube s'étendant à travers celui-ci et une partie inférieure définissant l'ouverture, le dispositif de collecte d'urine étant configuré pour être horizontal pendant l'utilisation avec le corps perméable aux fluides et la partie inférieure de la barrière imperméable aux fluides étant horizontaux ; et
l'extrémité du tube positionnée dans la chambre inclut une extrémité biseautée avec l'ouverture de tube positionnée au niveau de l'extrémité biseautée et le tube forme un angle par rapport à la partie distale du corps perméable aux fluides à partir de l'extrémité biseautée vers l'orifice et en dehors de la chambre.

13. Dispositif de collecte d'urine selon l'une quelconque des revendications 1 à 12, dans lequel la partie distale du corps perméable aux fluides recouvre l'ouverture de tube.

14. Dispositif de collecte d'urine selon l'une quelconque des revendications 1 à 13, dans lequel le corps perméable aux fluides inclut une saillie sur la partie distale du corps perméable aux fluides à proximité de la région d'extrémité distale de la barrière imperméable aux fluides et une poche (318) définie entre la saillie et la partie distale du corps perméable aux fluides, l'ouverture de tube et l'extrémité du tube étant positionnées à l'intérieur de la poche.

15. Procédé de collecte d'urine, le procédé comprenant :
l'insertion d'un pénis à travers une ouverture (206) dans une couche inférieure d'une barrière (202) imperméable aux fluides d'un dispositif de collecte d'urine, selon l'une quelconque des revendications 1 à 14, dans une chambre (204) du dispositif de collecte d'urine au moins partiellement définie par la barrière imperméable aux fluides, dans lequel l'ouverture (206) se trouve sur la couche inférieure, dans lequel l'ouverture est positionnée entre la région d'extrémité proximale et la région d'extrémité distale de la partie inférieure de la barrière imperméable aux fluides ;
l'aspiration de l'urine évacuée par le pénis dans la chambre à travers deux bras (211a,b ; 311a,b ; 411a,b ; 511a,b ; 611a,b ; 661a,b ; 711a,b ; 811a,b ; 861a,b) d'un corps (210, 310, 410, 510, 610, 660, 710, 810, 860) perméable aux fluides jusqu'à une partie distale (213, 313, 413, 513, 613, 663, 713, 813, 863) du corps perméable aux fluides au moins partiellement positionnée à proximité d'une région d'extrémité distale de la barrière imperméable aux fluides, les deux bras étant espacés l'un de l'autre et s'étendant à partir de la partie distale du corps perméable aux fluides ; et
l'aspiration de l'urine à partir de la partie distale du corps perméable aux fluides à travers un tube (208).
